# EUROPEAN PATENT SPECIFICATION

(11) **EP 4 606 423 B1**
(45) Date of publication and mention of the grant of the patent: **24.06.2026**
(21) Application number: 25158380.3
(22) Date of filing: 17.02.2025
(51) Int. Cl.: A61N 2/02, A61N 1/40, A61D 99/00

(54) **PULSED ELECTROMAGNETIC FIELD THERAPY DEVICE FOR CELLULAR REGENERATION AND PAIN MANAGEMENT**
GERÄT ZUR THERAPIE MIT GEPULSTEN ELEKTROMAGNETISCHEN FELDERN ZUR ZELLREGENERATION UND SCHMERZBEHANDLUNG
DISPOSITIF DE THÉRAPIE PAR CHAMP ÉLECTROMAGNÉTIQUE PULSÉ POUR LA RÉGÉNÉRATION CELLULAIRE ET LE TRAITEMENT DE LA DOULEUR

(30) Priority: 26.02.2024 LU 506459
(43) Date of publication of application: 27.08.2025
(73) Proprietor: Novahealthlux Holding S.à.r.l., 3317 Bergem (LU)
(72) Inventor: KLINKERT, Frank, 3317 Bergem (LU)
(74) Representative: Boncea, Oana-Laura

(56) References cited:
- WO-A1-2019/195816
- DE-B3- 102019 103 462
- GB-A- 2 602 669
- US-A- 5 267 938
- US-A1- 2010 087 699
- US-A1- 2014 249 355
- US-A1- 2016 184 601
- US-A1- 2017 001 030
- US-A1- 2023 285 768
- US-A1- 2024 042 226
- US-B1- 8 137 259

## Description

### TECHNICAL FIELD

The present invention relates to a pulsed electromagnetic field therapy (PEMFT) device for promoting cellular regeneration and pain management in animals. This device utilizes high-energy, short-duration pulses to penetrate deep tissues and directly stimulate cellular processes.

### BACKGROUND OF THE INVENTION

There exists a number of medical conditions in animals requiring preventive, post-traumatic and regenerative therapy, such as rideability problems in horses, tendon and ligament injuries, back and general muscular tension, osteoarthritis and arthritis, wounds and scars, and inflammations of all kinds. Also, diagnostics is necessary to recognize hidden pain processes in animals.

Magnetic medical aid used for treatment of varied medical conditions in human for a long time, recently has also become popular in treating animals, where pulsed electromagnetic field therapy (PEMFT) is utilized as a specific form of electrotherapy that uses pulsed electromagnetic fields at differing frequencies to provide a physiological effect.

The field of bioelectric stimulation and therapy has witnessed significant advancements in recent years, driven by the growing understanding of the role of electromagnetic fields in influencing cellular behavior and physiological processes. Bioelectric stimulation, also known as electromedicine or electrotherapy, involves the application of electromagnetic fields to stimulate biological tissues and promote healing. This approach has gained recognition for its potential to enhance tissue regeneration, alleviate pain, and accelerate recovery in various medical conditions.

Current PEMFT devices commonly use low-frequency, long-duration pulses, resulting in muscle contractions without necessarily targeting cellular regeneration. Document EP 3710106 B1 discloses a PEMFT device with a coil loop inductor, which provides a low-frequency magnetic field therapy. However, the very strong muscle contractions in the low-frequency therapy by the known device are not an indicator of cell regeneration, but result from a very prolonged pulse that generates a long-lasting current in the coil. This current causes the noticeable muscle contractions. Also, detection of reaction points is not possible with the known device.

One of the key principles underlying bioelectric stimulation is the modulation of cellular activity through the manipulation of electrical signals. Cells in the body communicate with each other and respond to external stimuli through electrical impulses. These signals play a crucial role in regulating cellular functions such as growth, proliferation, and differentiation. By harnessing the power of electromagnetic fields, researchers and clinicians have sought to influence these electrical signals to promote healing and restore normal physiological function.

The concept of using electromagnetic fields for therapeutic purposes dates back centuries, with historical accounts documenting the use of magnets and electrical devices for treating various ailments. However, it wasn't until the 20th century that scientific research began to shed light on the mechanisms underlying bioelectric stimulation. The development of technologies such as transcutaneous electrical nerve stimulation (TENS) and pulsed electromagnetic field therapy (PEMFT) paved the way for more targeted and effective approaches to electrotherapy.

PEMFT, in particular, has emerged as a promising modality for promoting tissue regeneration and relieving pain. Unlike continuous electromagnetic fields, which may generate excessive heat and tissue damage, pulsed electromagnetic fields deliver short bursts of energy at specific frequencies, minimizing the risk of adverse effects. This precise modulation of electromagnetic signals allows for targeted stimulation of cells and tissues, making PEMFT a versatile tool in clinical practice.

The therapeutic potential of PEMFT extends to a wide range of medical applications, including orthopedics, sports medicine, wound healing, and neurology. Research studies have demonstrated its efficacy in accelerating bone repair, reducing inflammation, and improving circulation. Furthermore, PEMFT has shown promise in managing chronic pain conditions such as osteoarthritis, fibromyalgia, and neuropathic pain, offering patients a non-invasive alternative to traditional treatments.

In recent years, advancements in technology have led to the development of portable PEMFT devices that provide clinicians and patients with greater flexibility and convenience in therapy delivery. These devices leverage innovative designs and sophisticated algorithms to deliver targeted electromagnetic stimulation to specific areas of the body. By incorporating features such as user-friendly interfaces, customizable treatment protocols, and real-time monitoring capabilities, portable PEMFT devices empower healthcare providers to optimize therapy outcomes while ensuring patient comfort and compliance.

In the quest for enhancing bioelectric stimulation and therapeutic applications, various inventions have contributed to the evolution of the field. Notable among them are:
1. US20190054308A1:
   This patent discloses systems and techniques for applying a pulsed electromagnetic field to the body. The described device is a self-contained, portable pulsed electromagnetic field generator designed to administer electromagnetic pulses to the feet and hands. The device consists of an electromagnetic field generator providing power, a device base receiving pulses from the feet, and a device bulb receiving pulses from the hands. The objective is to increase the energy of cells in the body through this electromagnetic stimulation.
2. US6786859B2:
   This invention discloses an apparatus designed for applying magnetic field treatment to a biological entity. The apparatus includes a signal generation unit responsible for generating an electrical treatment signal. An induction coil mat, connected to the signal generation unit, generates a magnetic field corresponding to the electrical treatment signal. The disclosed electrical treatment signal incorporates frequency components of approximately 300 Hz, 600 Hz, 800 Hz, and 1,000 Hz in superposition. Additionally, a set of further frequencies ranging from 2 Hz to 32 Hz can be added, selected based on the desired treatment mode of operation.
3. DE102019103462B3 discloses a portable PEMFT device, which is a high-voltage therapy device with a voltage of, for example, 30,000 V, a current of, for example, 4,000 A and an oscillation frequency of, for example, 200 kHz. The device comprises a housing, a capacitor comprised in the housing, and a treatment part which has a treatment loop arranged outside the housing and electrical power cables which can be connected to the capacitor. The treatment part is detachably connected to the housing via a connecting unit comprising an electrically actuated safety element that can be adjusted between a locking position and a removal position, so as to provide a possibility of detachment of the treatment part only when the electrical energy content of the energy storage device is within a defined value range. The safety element is controlled via a control unit in the therapy device.

While these prior arts have contributed valuable insights into bioelectric stimulation, the device distinguishes itself by offering a unique combination of targeted stimulation and diagnostic capabilities. Its high-energy pulse field and non-invasive delivery mechanism make it a versatile tool for therapeutic applications, with an added diagnostic capability to identify hidden pain processes early. The device, with its innovative components and features, seeks to redefine the landscape of bioelectric stimulation for enhanced healthcare outcomes.

One such portable PEMFT device is the device, which represents a breakthrough in bioelectric stimulation and animal therapy. Designed to penetrate deep into the body, the device delivers high-energy pulse fields that stimulate cellular regeneration and alleviate pain. Its non-invasive and non-contact delivery method makes it suitable for a wide range of medical conditions, including musculoskeletal disorders, inflammatory conditions, and neurological disorders. Moreover, the device's diagnostic capabilities enable early detection of hidden pain processes, facilitating timely intervention and improved patient outcomes.

In conclusion, bioelectric stimulation and therapy, particularly through PEMFT, offer promising avenues for enhancing healing and improving quality of life for patients. With continued research and innovation, portable devices like the device have the potential to revolutionize the field of electromedicine, providing clinicians and patients with effective, convenient, and safe treatment options for a variety of medical conditions.

Therefore, it would be desirable to overcome the aforementioned drawbacks. Particularly, there is a need to develop a PEMFT device for treating animals, which would have a short effect on the organism while ensuring healing through protein formation in affected cells, and which also would be useful in diagnostics so as to recognize hidden pain processes at an early stage and thus provide successful treatment thereof. Also, there is a need to provide a maximal safety of the device for users. Furthermore, it is desirable to protect the device from impact of external environment so as to extend its service life. Additionally, it is desirable to provide a possibility of simple and quick adjustment of the operational parameters of the device during the use.

### SUMMARY OF THE INVENTION

In order to effectively solve the above-mentioned problems, the present invention provides a portable PEMFT device with an intuitive user interface, which is designed for treating animals like dogs, cats, horses, and other animals of similar size and which uses repetitive peripheral magnetic pulse stimulation (RPMPS) to deliver short, strong magnetic fields in the microsecond range. This approach avoids muscle contractions while directly targeting cellular processes associated with regeneration.

The present invention relates to a novel bioelectric stimulation device, designed to deliver targeted electromagnetic stimulation for therapeutic and diagnostic purposes. The device utilizes high-energy pulse fields to penetrate deep into the body, stimulating cellular regeneration and alleviating pain. In addition to its therapeutic applications, the device offers diagnostic capabilities for early detection of hidden pain processes, enabling timely intervention and improved patient outcomes.

One of the key features of the device is its ability to harness the bioelectric spectrum of action, which reaches up to 20 cm deep into the body. This extensive reach allows the device to work directly in every single cell of the body, where regeneration occurs. By penetrating deep into tissues, the device can stimulate cellular activity and promote healing at the cellular level. The device achieves this through the generation of high-energy pulse fields, which induce bioelectric effects that stabilize cell membrane potential and facilitate normal cellular function.

The device is primarily designed for therapeutic use in animals, offering a non-invasive and non-contact approach to bioelectric stimulation. The device can be used to treat a variety of medical conditions, including musculoskeletal disorders, inflammatory conditions, and neurological disorders. By delivering targeted electromagnetic stimulation to specific areas of the body, the device promotes tissue regeneration, reduces inflammation, and relieves pain.

The therapeutic effects of the device are achieved through repetitive peripheral magnetic pulse stimulation, which delivers short, strong magnetic fields in the microsecond range. These pulses stimulate cellular activity and enhance tissue repair processes, leading to improved healing outcomes. Unlike low-frequency therapy devices that generate strong muscle contractions, the device's high-frequency pulses have a short duration and exert a direct effect on cellular function without causing noticeable muscle contractions.

In addition to its therapeutic applications, the device can also be used for diagnostic purposes. The device is capable of detecting hidden pain processes, such as tension or inflammation, at an early stage. By analyzing the body's response to electromagnetic stimulation, the device can identify areas of abnormal cellular activity and provide valuable insights into underlying health conditions. This diagnostic capability enables healthcare providers to intervene proactively and initiate appropriate treatment strategies to address pain and inflammation.

The present invention is set out in the appended set of claims.

The device is a sophisticated piece of equipment designed to provide targeted bioelectric stimulation and diagnostic capabilities. It consists of several integral components that work in harmony to deliver therapeutic benefits and ensure accurate diagnostic results.

Particularly, the present invention is directed to a portable PEMFT device for animal treatment, comprising:
- a durable plastic housing containing: a high voltage energy storage capacitor configured to store energy used in generating magnetic pulses; a magnetic coil configured to generate the magnetic pulses for therapeutic purposes; a high voltage power supply unit configured to deliver energy to the capacitor; a high voltage switch configured to control release of high voltage energy for the magnetic pulse generation; a high voltage shielding configured to prevent electromagnetic interference (EMI); a flyback diode to prevent damage of the device from voltage spikes; a user interface configured to control operation of the device; a controller configured to regulate generation and delivery of the magnetic pulses; and an AC/DC power supply unit; and
- a detachable power cord configured to connect the device to an AC power supply via an appliance coupler to provide power for the device operation.

The high voltage shielding encloses the high voltage energy storage capacitor, the high voltage power supply unit, the high voltage switch, the flyback diode and the controller.

The housing serves as a protective casing, enclosing and safeguarding the internal components from external damage; it is constructed from durable materials to withstand the rigors of clinical use while providing a safe environment for the device's operation.

The user interface is designed to be intuitive and user-friendly, allowing healthcare providers to easily navigate through various settings and parameters; it enables customization of treatment protocols based on patient needs and allows real-time monitoring of therapy sessions; the interface preferably is configured to provide a real-time feedback on treatment parameters, patient response, and/or diagnostic results, empowering healthcare professionals to make informed decisions during treatment.

The high-voltage shielding ensures the accurate delivery of pulse fields to the target area; this shielding helps maintain the integrity of the electromagnetic signals, minimizing distortion and maximizing the effectiveness of the therapy.

The controller is responsible for regulating operation of the device; it controls various parameters such as pulse frequency, intensity, and duration, allowing for precise modulation of electromagnetic stimulation; the controller ensures optimal therapeutic outcomes by adjusting treatment parameters to suit individual patient needs and response.

The flyback diode that protects the internal circuitry of the device from voltage spike, prevents damage to sensitive components by diverting excess voltage away from the circuit, ensuring safe and reliable operation of the device.

The AC/DC power supply unit is integrated into the device to convert AC voltage to the appropriate DC voltage required for operation; it is compliant with safety standards and regulations, providing a stable and consistent power source for the device.

The high voltage (HV) power supply unit is responsible for generating the energy required for pulse field stimulation, delivering precise electromagnetic pulses to the target area, stimulating cellular activity and promoting tissue regeneration; the HV power supply unit ensures accurate and reliable delivery of therapeutic benefits to the patient.

The high voltage energy storage capacitor is provided to store electrical energy and release it in short bursts; this capacitor plays a crucial role in producing pulse fields for therapeutic purposes, ensuring consistent and controlled stimulation of the target area.

The high voltage switch controls the timing and duration of pulse fields generated by the device; it allows for precise modulation of electromagnetic stimulation, enabling healthcare providers to tailor treatment protocols to individual patient needs.

The magnetic coil delivers pulse fields to the target area, stimulating cellular activity and promoting tissue regeneration; the magnetic coil is designed to provide consistent and uniform electromagnetic stimulation for optimal therapeutic outcomes.

Overall, the device represents a state-of-the-art solution for targeted bioelectric stimulation and diagnostics, providing healthcare providers with the tools they need to deliver effective and personalized care to their patients.

According to one preferred embodiment, an input voltage provided by the AC/DC power supply unit is 100-240 V and a line frequency provided by the AC/DC power supply unit is 50/60 Hz.

According to another preferred embodiment, a maximum voltage generated by the high voltage power supply unit is 15000 V.

According to one preferred embodiment, the high-voltage switch is configured to short-circuit the charged high voltage capacitor at a rate of about 1-2 Hz to ensure the flow of current through the magnetic coil for generation of the magnetic pulses.

According to one more preferred embodiment, the device is configured to provide a high frequency magnetic field having a frequency in the range of from about 115 to about 135 kHz.

According to one more preferred embodiment, the device is configured to provide a magnetic flux density of from about 200 to about 1000 G.

According to another preferred embodiment, a maximum current in the magnetic coil is 4000 A.

According to another preferred embodiment, the user interface includes controls for adjusting pulse frequency, intensity, and duration.

According to another preferred embodiment, the user interface is configured to enable selection between three parameter modes selectable depending on an animal species to be treated, preferably a first mode of the three parameter modes sets treatment parameters for horses and other animals of similar size, a second mode of the three parameter modes sets treatment parameters for dogs and other animals of similar size, and a third mode of the three parameter modes sets treatment parameters for cats and other animals of similar size, more preferably the operation parameters of the first mode comprise generation of high voltage of 15 kV, the operation parameters of the second mode comprise generation of high voltage of 9 kV, and the operation parameters of the third mode comprise generation of high voltage of 6 kV.

According to one more preferred embodiment, the high voltage shielding is made of perforated metal sheets, wherein the metal is selected from copper, brass, nickel, silver, steel, and tin, and preferably, the HV shielding is made of perforated stainless-steel sheets.

According to one more preferred embodiment, the magnetic coil is provided in the form of a cable loop preferably made of a high voltage cord being a flexible tin-plated copper conductor covered by a co-extruded semiconductor layer, a high-grade silicon rubber insulation, and a PUR varnished synthetic fiber braid for enhanced friction resistance, with two ends of the high-voltage cord, which do not form a loop part of the cable loop, connecting to a high-voltage section of the device, the cable loop preferably configured to be placed in a free space of the housing outside the HV shielding when not in use, with the loop part configured to be contacted by an operator and taken out of the housing for performing therapy in respect of an animal in need.

According to one more preferred embodiment, the device further comprises two wheels and a handle which are attached to the housing for portability, preferably the handle is a telescopic handle.

According to one more preferred embodiment, the device further comprises at least one fan provided in the housing for ventilation purpose.

According to one more preferred embodiment, the housing has a cover pivotally connected to a top part of the housing and preferably lockable to the housing with the help of at least one latch.

According to one more preferred embodiment, the device further comprises a display, preferably a touch display, to display the user interface, wherein the display is preferably provided on an inner surface of the cover.

In contrast to the existing PEMFT devices using low-frequency, long-duration pulses, resulting in muscle contractions without necessarily targeting cellular regeneration, the present invention employs high-frequency, short-duration pulses to create a bioelectric spectrum of action reaching 20cm deep, directly influencing cellular function. Short, strong magnetic fields in the microsecond range are delivered to the animal body via the treatment cable loop. The pulses from the device advantageously have an extremely short effect on the animal organism.

The device according to the present invention is 100% pain-free and drug-free, non-invasive and yet highly effective.

The device according to the present invention can be used by doctors, therapists and specially trained personnel for preventive, post-traumatic and regenerative purposes to optimize the recovery process and the animal's well-being.

The device according to the present invention can also be effectively used in diagnostics. Hidden pain processes in particular, such as tension or inflammation, are recognized at an early stage with the help of the device according to the present invention and can therefore be treated successfully.

The device according to the present invention is safe for operators and animals by providing HV shielding and loop multiple strong insulation, is durable, dirt- and water-resistant to optimize it for stables.

Furthermore, the device according to the present invention has user-friendly interface provided by a touch display.

The operation of the device involves a series of steps designed to deliver targeted bioelectric stimulation and diagnostic capabilities effectively. From setup to treatment, each stage of operation plays a crucial role in ensuring optimal therapeutic outcomes and diagnostic accuracy.

Before initiating treatment or diagnostics with the device, healthcare providers must first ensure proper setup and preparation. This includes positioning the device near the target area of the body and preparing the patient for therapy or evaluation. The device should be placed in a stable location, and the magnetic coil should be positioned in close proximity to the skin surface, ensuring optimal delivery of electromagnetic pulses. Additionally, the user interface should be configured to the desired treatment parameters, including pulse frequency, intensity, and duration. Once the device is properly positioned and configured, it can be activated to begin therapy or diagnostics. The activation process initializes the device's internal components, including the controller, power supply, and magnetic coil. This ensures that the device is ready to deliver electromagnetic stimulation and analyse the body's response effectively.

The core function of the device is to deliver electromagnetic pulses to the target area of the body. These pulses are generated by the magnetic coil and are delivered in short bursts to penetrate deep into tissues. The pulses are designed to stimulate cellular activity and promote healing at the cellular level. By delivering targeted electromagnetic stimulation, the device can promote tissue regeneration, reduce inflammation, and relieve pain effectively.

During therapy or diagnostics, the device provides real-time monitoring of treatment parameters and patient response. The user interface displays feedback on pulse frequency, intensity, and duration, allowing healthcare providers to monitor the progress of therapy and make adjustments as needed. For example, if the patient experiences discomfort or adverse effects, therapy settings can be modified to ensure patient safety and comfort.

In diagnostic mode, the device analyses the body's response to electromagnetic stimulation to identify areas of abnormal cellular activity. By detecting changes in tissue response, such as increased sensitivity or inflammation, the device can provide valuable insights into underlying health conditions. This analysis enables healthcare providers to diagnose hidden pain processes and inflammation at an early stage, facilitating timely intervention and treatment.

Throughout the operation, the device collects data on treatment parameters, patient response, and diagnostic findings. This data is stored in the device's memory and can be accessed by healthcare providers for further analysis and reporting. By documenting treatment outcomes and diagnostic results, the device helps healthcare providers track patient progress and adjust treatment strategies as needed.

Once therapy or diagnostics are complete, the device can be deactivated and shut down. This process involves powering off the device and ensuring that all internal components are properly stored and secured. Deactivation and shutdown help prolong the lifespan of the device and ensure its safe storage until the next use.

### BRIEF DESCRIPTION OF THE DRAWINGS

The present invention will be described more fully hereinafter with reference to the accompanying drawings, in which prominent aspects and features of the invention are illustrated.
**Fig. 1** is a block diagram of main functional sections of a PEMFT device according to an embodiment of the present invention.
**Fig. 2** is a block diagram of equipment of the PEMFT device according to an embodiment of the present invention including peripherals and auxiliary equipment used.
**Fig. 3** is a perspective view of the PEMFT device according to an embodiment of the present invention with a cover of a housing opened, a cable loop taken out and a telescopic handle extended.
**Fig. 4** is a side view of the PEMFT device shown in Fig. 3 with the cover of the housing opened, the cable loop put inside the housing and the telescopic handle folded.
**Fig. 5** is a back view of the PEMFT device shown in Fig. 3 with the cover of the housing closed and the telescopic handle folded.
**Fig. 6** is another side view of the PEMFT device shown in Fig. 3 with the cover of the housing closed and the telescopic handle folded.
**Fig. 7** is a user interface on a display of the PEMFT device shown in Fig. 3, which shows three operation modes.
**Fig. 8** is a perspective view of the PEMFT device shown in Fig. 3 with the cover of the housing opened and a front wall of the housing removed to show internal equipment arrangement.
**Fig. 9** is the perspective view shown in Fig. 8 with front, back and top parts of a HV shielding removed to show equipment arrangement inside the HV shielding.
**Figs. 10-12** show the results of conducted emission tests of the PEMFT device according to the present invention.
**Figs. 13-15** show the results of radiation emission tests of the PEMFT device according to the present invention.

The present figures relate to schematic drawings so that any dimension of the elements shown in the drawings may deviate from a specifically implemented setup.

### DETAILED DESCRIPTION

The present invention is now described in more details with reference to non-limiting examples. For example, modifications of specific elements of the preferred embodiments described hereafter may be combined with other modifications so as to provide further embodiments of the present invention.

The present invention is directed to a PEMFT device that is suitable for use on animal creatures such as dogs, cats, horses and animals of similar size suffering from injury, disease, restricted movement due to pain and acute and chronic inflammatory processes of the musculoskeletal system.

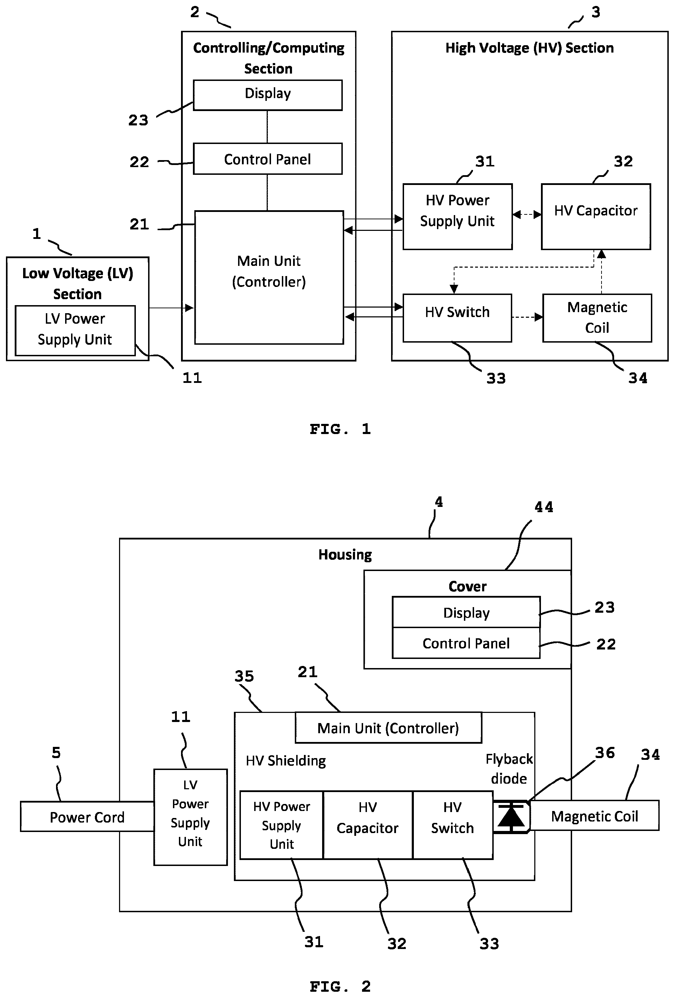

Figure 1 illustrates a block diagram of main functional sections of the PEMFT device according to an embodiment of the present invention. The PEMFT device comprises a low voltage (LV) section 1, a controlling/computing section 2 and a high voltage (HV) section 3.

The controlling/computing section 2 comprises a main unit (controller) 21, a control panel 22 and a display 23, the main unit 21 being connected with the control panel 22 and the control panel being connected with the display 23. Particularly, the main purpose of the controlling/computing section 2 is providing a user interface and a control of the HV section 3. The secondary purpose of the controlling/computing section 2 is monitoring the HV section 3, monitoring the LV section 1, error detection, error management and if required, provision of 5VDC. The main unit (controller) 21 particularly governs pulse generation and treatment protocol execution.

The LV section 1 is connected to the controlling/computing section 2, and particularly to the main unit 21. The main purpose of the LV section 1 is to provide power to the controlling/computing section 2 and to the HV section 3. The LV section 1 comprises a 24VDC power supply unit 11 (input voltage is 100-240 V (50/60 Hz)). For cost reasons, the 5VDC can also be derived from the 24VDC.

The HV section 3 comprises a high voltage (HV) power supply unit 31, a high voltage energy storage (a high voltage (HV) capacitor) 32, a high voltage (HV) switch 33 and a magnetic coil 34. The HV section 3 is provided for generating an alternating magnetic field by cyclically discharging the HV capacitor 32 via the magnetic coil 34.

The main unit 21 of the controlling/computing section 2 is connected with the HV power supply unit 31 and with the HV switch 33.

The HV section 3 functions as follows. The HV power supply unit 31 generates a high voltage (up to 15000 V) that is required for pulse generation. The generated high voltage is used to charge the HV capacitor 32. The HV capacitor 32 stores the charging energy for high-voltage pulse transmission. The HV switch 33, which is used to control the release of high-voltage energy for pulse generation, short-circuits the charged HV capacitor 32 at a specified rate (about 1-2 Hz) and thus ensures the flow of current through the magnetic coil 34. The magnetic coil 34 generates and transmits a pulsed magnetic field through the winding when the current flows through. The pulsed magnetic field is used for treatment.

The PEMFT device provides a high-frequency magnetic field that may have a frequency in the range of from about 115 to about 135 kHz.

The PEMFT device can provide a magnetic flux density of from about 200 to about 1000 G (about 20 - 100 mT).

The current in the magnetic coil 34 can reach up to 4000 A.

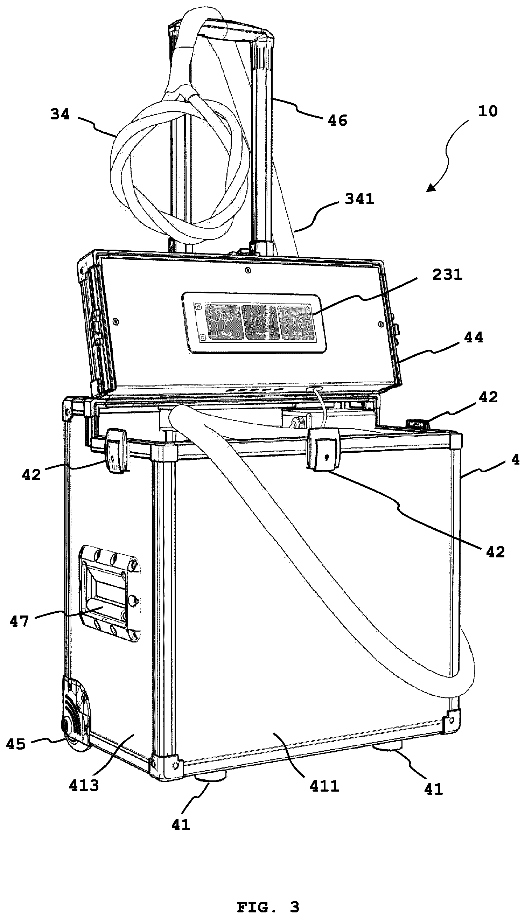

Figure 3 shows a perspective view of the PEMFT device 10. The device 10 is portable. It comprises a housing 4 that protects internal components and provides user interface mounting, as will be explained in details below.

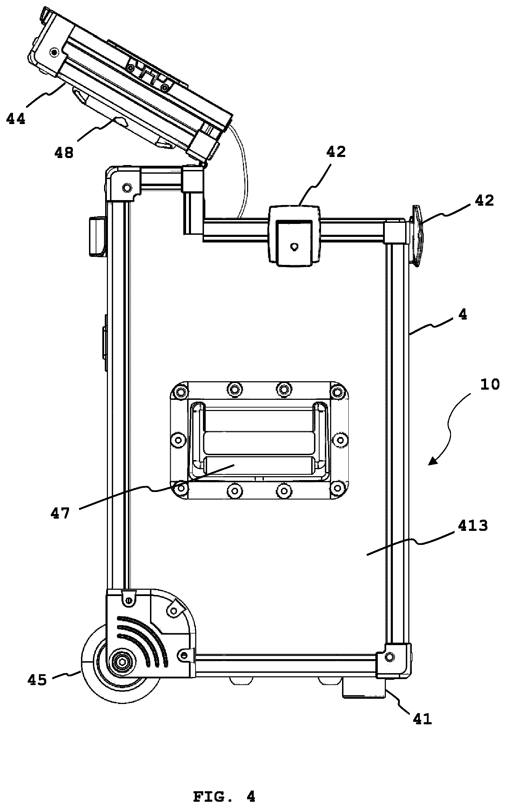

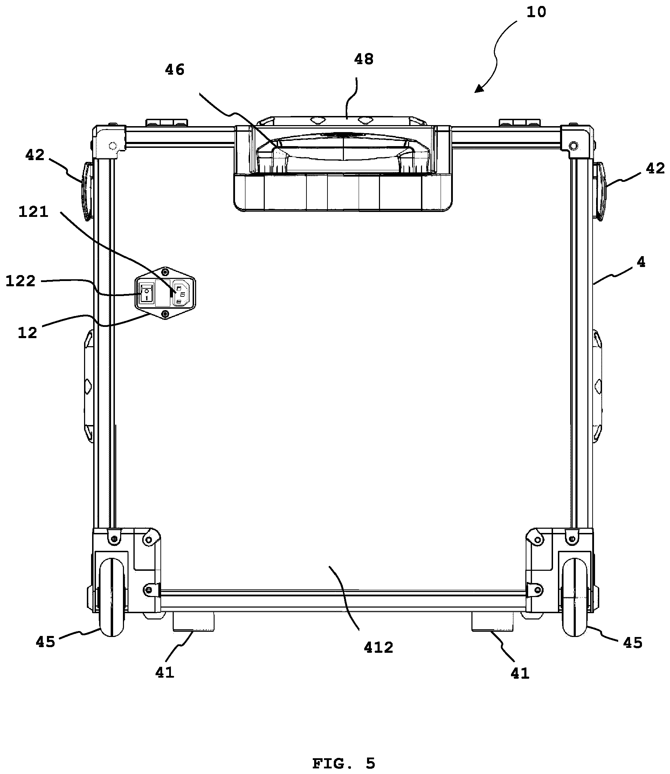

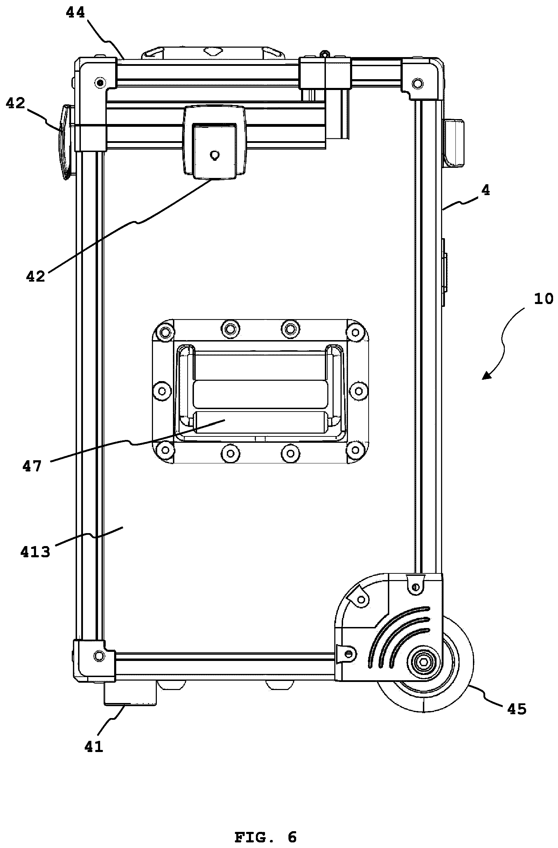

Two wheels 45 are attached to the housing 4 for portability. Preferably, the wheels 45 are mounted distantly from each other at an edge between the bottom wall and a back wall of the housing 4. The back wall 412 (see also Figure 5) of the housing 4 is provided with a telescopic handle 46 having a button (not shown) adapted to activate folding or extending of the telescopic handle 46. As shown in Figures 4-6, when the telescopic handle 46 is folded it is hidden inside the housing 4 along an internal surface of the back wall 412 of the housing 4. The wheels 45 and the telescopic handle 46 are intended for easier transportation when the device 10 is not in use. The housing 4 is also provided with two legs 41 mounted distantly from each other to the bottom wall of the housing 4 closer to a front wall 411 of the housing 4 to support the device 10 when not transported. Additionally, a carry handle 47 can be mounted on each of two side walls 413 of the housing 4, as seen in Figures 4 and 6. In other possible embodiments, the housing can comprise only one carry handle 47 mounted on one of the two side walls 413 or can be provided without any carry handles.

The housing 4 has a cover 44 pivotally connected to the top part of the housing 4 to enable partial opening of the housing 4 when the cover 44 is lifted in direction of the back wall 412 of the housing 4. When the cover 44 is closed, it forms a top wall of the housing. The closed cover 44 can be locked with the help of three latches 42, one provided on a top edge of one side wall 413, one provided on a top edge of another side wall 413 and one provided on a top edge of the front wall 411 of the housing 4. However, the number of the latches 42 can be different from three (for example, one, two or four latches). Also, any other locking mechanisms can be used instead of latches.

The entire device 10 is supplied by AC supply voltage via a power inlet/switch assembly 12 mounted on the back wall 412 of the housing 4 (as shown in Figure 5) with a detachable power supply cord 5 (L, N, PE) with EU mains plug. The power cord 5 connects the device 10 to a standard AC power outlet. The power inlet/switch assembly 12 has an ON/OFF switch 122 mounted near an appliance inlet 121 and two input high breaking fuses (not seen in the figures) incorporated within the power inlet/switch assembly 12. Inside the housing 4 of the device 10, there is a LV power supply unit 11, preferably IEC/EN 62368-1-1 approved AC/DC power supply unit (equivalent to 2 x MOOP), that is connected to the power inlet/switch assembly 12 and provides regulated power to the device 10.

The HV power supply unit 31, the HV capacitor 32, the HV switch 33 and the main unit 21 are arranged in the interior of the housing 4 within a HV shielding 35, as shown in Figures 2, 8 and 9. The LV power supply unit 11 is attached to one of the side shields 351 from the outside of the HV shielding 35.

The HV power supply unit 31 preferably is UL 60950-1 and IEC 62368-1 approved HV power supply unit, for example a HV power supply unit 15C24-P125 (Input: 23-30 Vdc; 6,5 A; 125 W; Output: 15 kV; 8,3 mA; 125 W) provided by ULTRAVOLT INC.

The HV capacitor 32 preferably is IEC/EN 60601-1 approved HV capacitor, for example, HV capacitor PSE11024 (30 kV DC, 0,30 µF) provided by Shanghai Pluspark Electronics Co., Ltd.

The HV switch 33 preferably is IEC/EN 60601-1 approved Behlke HV switch (for example, a fast high voltage thyristor switch HTS 200-800 SCR I-FWD, 20 kV DC; max. 8000 A DC; 70°C).

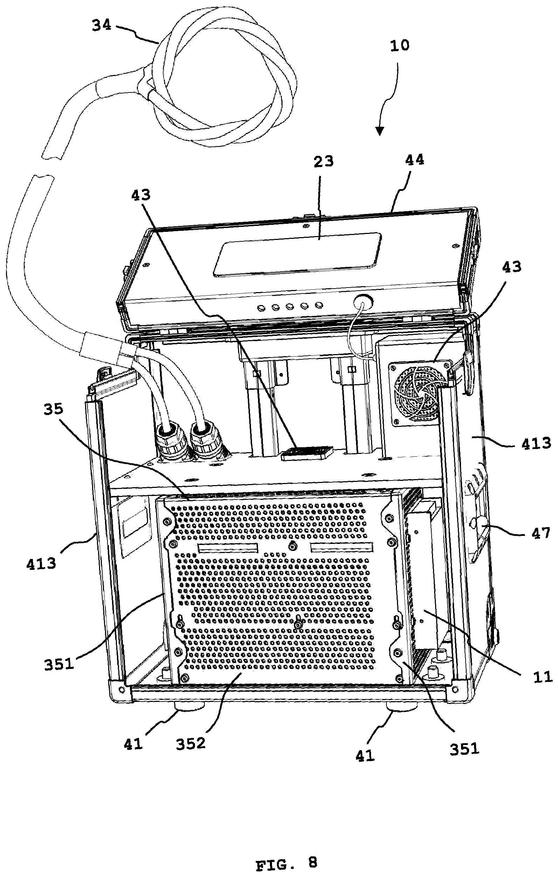

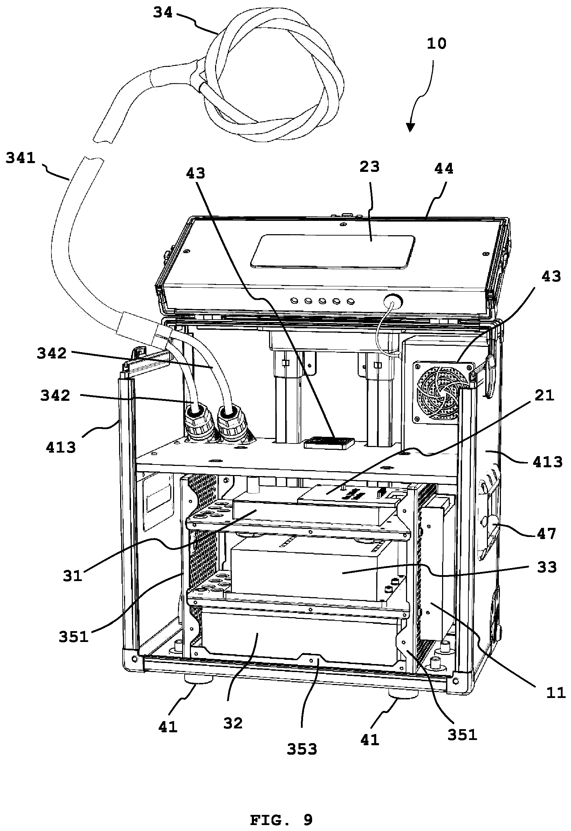

The HV shielding is made using materials that prevent electromagnetic interference. In one embodiment, the HV shielding is made of perforated metal sheets. Common sheet metals for shielding include copper, brass, nickel, silver, steel, and tin. Preferably, the HV shielding is made of perforated stainless-steel sheets. As shown in Figures 8 and 9, the HV shielding consists of a bottom shield 353, two side shields 351, a front shield 352, a back shield (not shown) and a top shield (not shown) to cover the HV components from all sides and ensure user safety by blocking high voltage radiation. In one of the embodiments, the front shield 352, the back shield and the top shield can be made as a single perforated metal plate, and the bottom shield 353 and the two side shields 351 are made as separate perforated metal plates, all of which are screwed together during assembling.

The HV capacitor 32 can be arranged on the bottom shield 353 of the HV shielding 35. The HV switch 33 can be arranged above the HV capacitor 32 on a supporting perforated plate made of the same material as the HV shielding. The HV power supply unit 31 can be arranged above the HV switch 33 on another supporting plate made of the same material as the HV shielding. The main unit 21 is attached to the HV power supply unit 31.

If any HV cable would touch the inner sides of the metal HV shielding 35, the HV capacitor 32 will discharge due because the shielding is grounded.

The device 4 is also equipped with a flyback diode 36 (shown in Figure 2) that protects circuitry from voltage spikes.

Two fans 43 are arranged in the housing 4 for ventilation purposes, as shown in Figures 8 and 9. In an alternative embodiment, the number of fans may be different from two, for example, one or three fans may be provided.

As can be seen in Figures 2, 3, 8 and 9, the magnetic coil 34 is an external component which is provided in the form of a cable loop placed in a free space of the upper part of the housing 4 above the HV shielding 35 when the housing 10 is closed and which can be directly contacted by an operator to take it out of the housing 4 and perform therapy in respect of an animal in need. For this purpose, the cable loop 34 is placed by the operator adjacent to, or around, a part of the animal body (such as a limb or joint) where the physiological effect of the pulsed electromagnetic field is desired.

A HV cord forming the cable loop 34 is provided with multiple strong insulation. Particularly, the HV cord for the cable loop 34 is a flexible tin-plated copper conductor covered by a co-extruded semiconductor layer, a high-grade silicon rubber insulation, and a PUR varnished synthetic fiber braid for enhanced friction resistance. A part of the HV cord forming the cable loop 34 is additionally covered by a heat shrink sleeve, and a part of the HV cord from the cable loop 34 to a point of separation of two ends 342 of the HV cord, which connect to the HV section, is additionally covered by a silicon tube 341.

Interaction with the device 10 to set treatment parameters is performed via a user interface 231 displayed on the display 23. The display 23 can be mounted on an inner side of the cover 44. Such arrangement prevents the display 23 from damaging during transportation of the device 10 or when the device 10 is not in use, while providing a direct accessibility to the display 23 when the cover 44 is opened during the use of the device 10. The control panel 22 is arranged in the cover 44 and is attached to a rear side of the display 23. Preferably, the display 23 can be adjusted to lighting conditions. Preferably, the display 23 is a touch display.

The functions of the device are monitored by means of the display 23. The user interface 231 displayed on the display 23 can indicate the status of the treatment (intensity, time, duration) as well as possible errors and enables a modular, stepless adjustment of working parameters. Preferably, the user interface 231 can provide a possibility of selection between three parameter modes selectable depending on an animal species to be treated. The three parameter modes preferably specify suitable treatment parameters for the following three categories of animals: 1) horses and other animals of similar size ("big-size" animals), 2) dogs and other animals of similar size ("middle-size" animals), and 3) cats and other animals of similar size ("small-size" animals). The three modes may be displayed in the form of buttons with schematic pictures (or photos) of a horse, a dog and a cat respectively so that an operator could quickly select a suitable mode by touching on the corresponding part of the display 23.

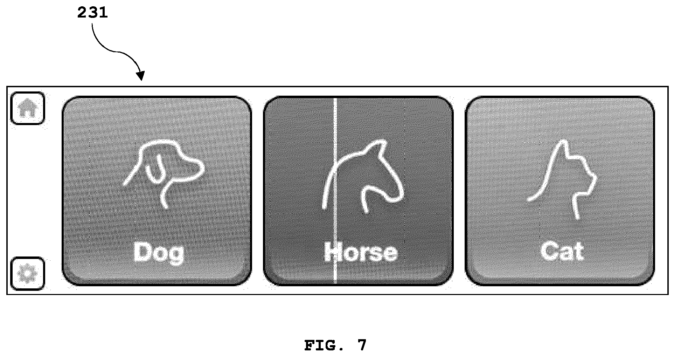

Alternatively, the three modes may be displayed in the form of buttons named "Horse", "Dog" and "Cat". In another embodiment, buttons may be displayed as a combination of the schematic picture (photo) and the name of the mode (as shown in Figure 7).

The touch display 23 as proposed in the invention is user-friendly and has an interactive and intuitive user interface 231, thereby enabling easy and quick use and control process with comfortable and pleasant impressions for an operator.

Preferably, the housing 4 of the device 10 is characterized by an ingress protection rating of IP 44. It means that the housing 4 is protected against the dirt and splash water, which optimizes the device 10 for stables and greatly extends its service life.

Preferably, the closed housing 4 of the device 10 is fireproof.

Preferably, the housing is made by non-metal durable material. Preferably, approved thermoplastic material can be used for these purposes. While the housing is made by non-metal material, the edges of the housing may be fixed together with metal parts and screws.

During the use of the PEMFT device according to the invention, the operator selects treatment parameters on the user interface 231. It is essential to select the application mode according to the animal species, such as "Horse", "Dog" or "Cat" mode, because depending on the selected animal species, the intensity of the therapy is graduated. The main unit (controller) 21 processes this information and triggers the HV power supply unit 31 to charge the HV capacitor 32. The HV switch 33 then releases the stored energy to the magnetic coil 34, generating a short, high-intensity magnetic pulse. This pulse penetrates deep tissues and interacts with cells, potentially promoting regeneration and pain relief.

Preferably, for performing therapy of horses or animals of similar size, the operation parameters of the mode "Horse" should be set to be about 15 kV. Preferably, for performing therapy of dogs or animals of similar size, the operation parameters of the mode "Dog" should be set to be about 9 kV. Preferably, for performing therapy of horses or animals of similar size, the operation parameters of the mode "Cat" should be set to be about 6 kV.

Preferably, the maximum application time should not exceed 20 minutes per application site.

Preferably, there should be a break of at least 4 hours between applications on the same animal. More preferably, there should be a single application on an animal in a need per day.

The PEMFT device according to the present invention was tested as a veterinary device and passed the medical standards Norm IEC 60601-1:2005, IEC 60601-1:2005/AMD1:2012, IEC 60601-1:2005/AMD2:2020, Norm IEC 60601-1-2:2014, IEC 60601-1-2:2014/AMD1:2020.

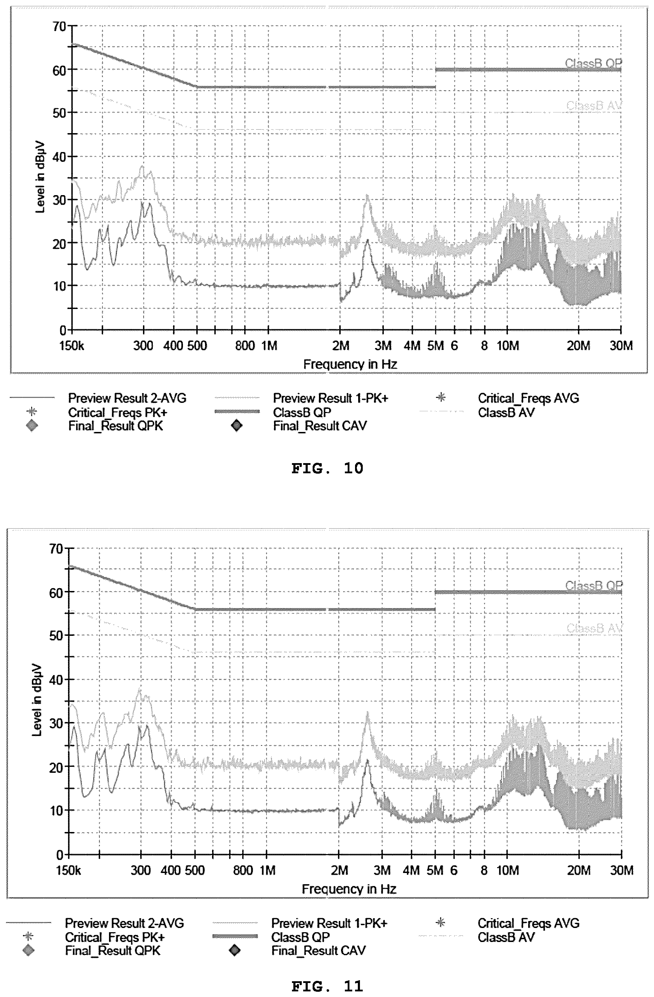

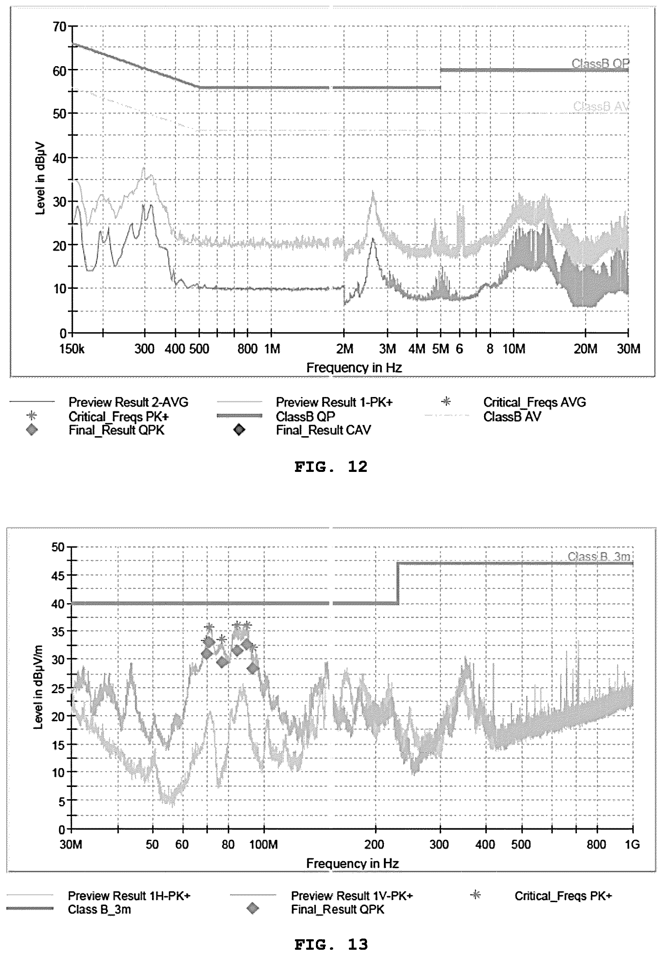

Results of conducted emission tests in accordance with the requirements for Class B equipment for conducted emissions are presented in Figures 10-12. Namely, Figure 10 shows the results under the operating mode Uin: 100 V / 50 Hz, "Horse" mode (15kV), Figure 11 shows the results under the operating mode Uin: 120 V / 60 Hz, "Horse" mode (15kV), and Figure 12 shows the results under the operating mode Uin: 230 V / 50 Hz, "Horse" mode (15kV). Measurement time for measurement was set to 50 ms, step size for measurement was set to 4 kHz, bandwidth was 9kHz, pre-amplifier was off. The test passed successfully. The result values did not exceed the quasi-peak and average class B limits.

According to the clinical studies, the PEMFT device according to the present invention does not damage cells. The bioelectric spectrum of action reaches up to 20 cm deep and thus works directly in every single cell of the animal body, where regeneration occurs. The cell membrane potential can stabilize again and enables the "power station" cell to work normally again. The therapy process performed by the device is absolutely pain-free for animals and highly effective in relation to its application on horses, dogs, cats, and other animals of similar weight. The device achieves healing through protein formation in affected cells.

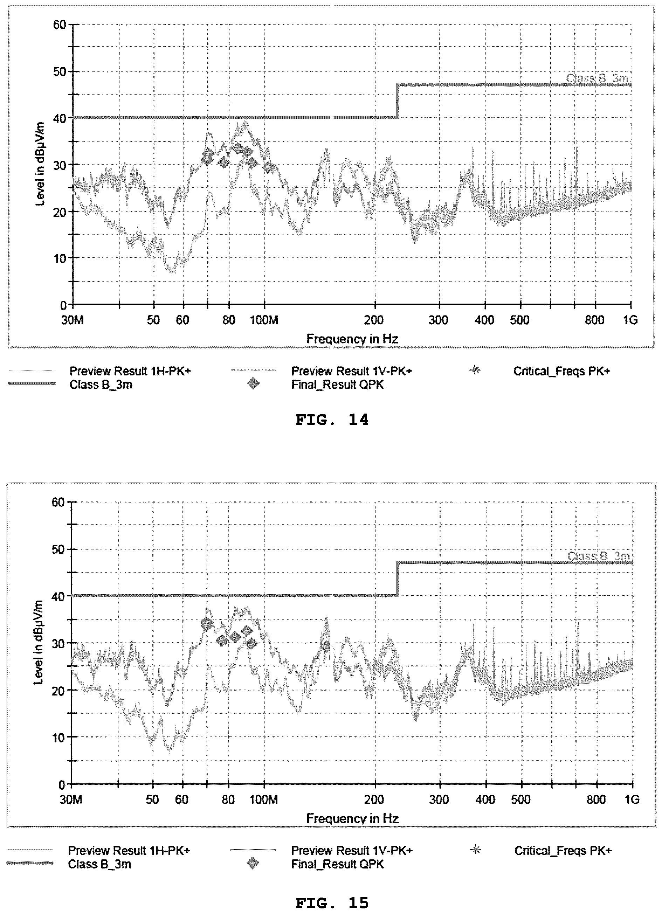

Results of radiation emission tests in accordance with the requirements for radiated emissions at frequencies up to 1000 MHz for Class B (3-m chambers) equipment are presented in Figures 13-15. Namely, Figure 13 shows the results under the operating mode Uin: 100 V / 50 Hz, "Horse" mode (15kV), Figure 14 shows the results under the operating mode Uin: 120 V / 60 Hz, "Horse" mode (15kV), and Figure 15 shows the results under the operating mode Uin: 230 V / 50 Hz, "Horse" mode (15kV). Measurement time for measurement was set to 5 ms, step size for measurement was set to 30 kHz, pre-amplifier was set to 20 dB. The test passed successfully. The result values did not exceed the class B limits.

The radiation of the PEMFT device according to the present invention is non-ionizing and is therefore far removed from, for example, harmful nuclear radiation.

Summarizing the above disclosure, the main advantages of the PEMFT device according to the present invention are:
- Deep tissue penetration (up to 20cm) for direct cellular stimulation.
- High-frequency, short-duration pulses specifically targeting cellular processes.
- Non-invasive and painless treatment experience for animals.
- Safety for operators and animals.
- Durable housing.
- Portable and user-friendly design for veterinary applications.

The above disclosed subject-matter is to be considered illustrative, and not restrictive, and serves to provide a better understanding of the present invention set out in the appended set of claims.

## Claims

1. A portable pulsed electromagnetic field therapy (PEMFT) device (10) for treatment of animals such as dogs, cats, horses and animals of similar size, comprising:
a housing (4) containing:
a high voltage energy storage capacitor (32) configured to store energy used in generating magnetic pulses;
a magnetic coil (34) configured to generate the magnetic pulses for therapeutic purposes;
a high voltage power supply unit (31) configured to deliver energy to the capacitor (32);
a high voltage switch (33) configured to control release of high voltage energy for the magnetic pulse generation;
a user interface (231) configured to control operation of the device (10);
a controller (21) configured to regulate generation and delivery of the magnetic pulses; and
an AC/DC power supply unit (11);
and
a detachable power cord (5) configured to connect the device (10) to an AC power supply via an appliance coupler to provide power for the device (10) operation,
the device (10) being **characterized in that** the housing (4) is a durable plastic housing and further comprises a flyback diode (36) to prevent damage of the device (10) from voltage spikes, and a high voltage shielding (35) configured to prevent electromagnetic interference and enclosing the high voltage energy storage capacitor (32), the high voltage power supply unit (31), the high voltage switch (33), the flyback diode (36) and the controller (21).

2. The portable PEMFT device (10) according to claim 1, wherein an input voltage provided by the AC/DC power supply unit (11) is 100-240 V and a line frequency provided by the AC/DC power supply unit (11) is 50/60 Hz.

3. The portable PEMFT device (10) according to claim 1 or claim 2, wherein a maximum voltage generated by the high voltage power supply unit (31) is 15000 V.

4. The portable PEMFT device (10) according to any one of the preceding claims, wherein the high-voltage switch (33) is configured to short-circuit the charged high voltage capacitor (32) at a rate of about 1-2 Hz to ensure the flow of current through the magnetic coil (34) for generation of the magnetic pulses.

5. The portable PEMFT device (10) according to any one of the preceding claims, wherein the device (10) is configured to provide a high frequency magnetic field having a frequency in the range of from about 115 to about 135 kHz.

6. The portable PEMFT device (10) according to any one of the preceding claims, wherein the device (10) is configured to provide a magnetic flux density of from about 200 to about 1000 G.

7. The portable PEMFT device (10) according to any one of the preceding claims, wherein a maximum current in the magnetic coil (34) is 4000 A.

8. The portable PEMFT device (10) according to any one of the preceding claims, wherein the user interface (231) includes controls for adjusting pulse frequency, intensity, and duration, and preferably is configured to provide a real-time feedback on treatment parameters, patient response to therapy, and/or diagnostic results.

9. The portable PEMFT device (10) according to any one of the preceding claims, wherein the user interface (231) is configured to enable selection between three parameter modes selectable depending on an animal species to be treated, wherein a first mode of the three parameter modes sets treatment parameters for horses and other animals of similar size, a second mode of the three parameter modes sets treatment parameters for dogs and other animals of similar size, and a third mode of the three parameter modes sets treatment parameters for cats and other animals of similar size, wherein the operation parameters of the first mode comprise generation of high voltage of 15 kV, the operation parameters of the second mode comprise generation of high voltage of 9 kV, and the operation parameters of the third mode comprise generation of high voltage of 6 kV.

10. The portable PEMFT device (10) according to any one of the preceding claims, wherein the high voltage shielding (35) is made of perforated metal sheets, wherein the metal is selected from copper, brass, nickel, silver, steel, and tin, wherein preferably, the HV shielding (35) is made of perforated stainless-steel sheets.

11. The portable PEMFT device (10) according to any one of the preceding claims, wherein the magnetic coil (34) is provided in the form of a cable loop (34) made of a high voltage cord being a flexible tin-plated copper conductor covered by a co-extruded semiconductor layer, a high-grade silicon rubber insulation, and a PUR varnished synthetic fiber braid for enhanced friction resistance, with two ends (342) of the high-voltage cord, which do not form a loop part of the cable loop (34), connecting to a high-voltage section of the device (10), the cable loop (34) configured to be placed in a free space of the housing (4) outside the HV shielding (35) when not in use, with the loop part configured to be contacted by an operator and taken out of the housing (4) for performing therapy in respect of an animal in need.

12. The portable PEMFT device (10) according to any one of the preceding claims, further comprising two wheels (45) and a handle which are attached to the housing for portability, preferably the handle is a telescopic handle (46).

13. The portable PEMFT device (10) according to any one of the preceding claims, further comprising at least one fan (43) provided in the housing (4) for ventilation purpose.

14. The portable PEMFT device (10) according to any one of the preceding claims, wherein the housing (4) has a cover (44) pivotally connected to a top part of the housing (4) and preferably lockable to the housing (4) with the help of at least one latch (42).

15. The portable PEMFT device (10) according to claim 14, further comprising a display (23), preferably a touch display, to display the user interface (231), wherein the display is provided on an inner surface of the cover (44).

## Patentansprüche

1. Ein tragbares PEMFT-Gerät (10) zur Behandlung von Tieren wie Hunden, Katzen, Pferden und Tieren ähnlicher Größe, umfassend:
ein Gehäuse (4), das enthält:
ein Hochspannungs-Energiespeicherkondensator (32), der zur Speicherung der zur Erzeugung magnetischer Pulse verwendeten Energie ausgelegt ist;
eine Magnetspule (34), die so konfiguriert ist, die magnetischen Pulse zu therapeutischen Zwecken zu erzeugen;
ein Hochspannungsnetzteil (31), das so konfiguriert ist, Energie an den Kondensator (32) zu liefern;
ein Hochspannungsschalter (33) konfiguriert, die Freisetzung von Hochspannungsenergie zur Erzeugung der magnetischen Pulse zu steuern;
eine Benutzeroberfläche (231), die so konfiguriert ist, den Betrieb des Geräts (10) zu steuern; ein Steuergerät (21), das so konfiguriert ist, die Erzeugung und Abgabe der magnetischen Pulse zu regeln; und
ein AC/DC-Netzteil (11);
und
ein abnehmbares Netzkabel (5), das das Gerät (10) über eine Geräteanschlusskupplung mit einer Wechselstromversorgung verbindet, um Strom für den Betrieb des Geräts (10) bereitzustellen,
das Gerät (10) **dadurch gekennzeichnet, dass** das Gehäuse (4) ein robustes Kunststoffgehäuse ist und ferner eine Freilaufdiode (36) aufweist, um das Gerät (10) vor Spannungsspitzen zu schützen, sowie eine Hochspannungsabschirmung (35), die so konfiguriert ist, elektromagnetische Störungen zu verhindern und den Hochspannungs-Energiespeicherkondensator (32), das Hochspannungsnetzteil (31), den Hochspannungsschalter (33), die Freilaufdiode (36) und das Steuergerät (21) umschließt.

2. Das tragbare PEMFT-Gerät (10) nach Anspruch 1, wobei eine Eingangsspannung, die vom AC/DC-Netzteil (11) bereitgestellt wird, 100-240 V beträgt und eine Netzfrequenz, die vom AC/DC-Netzteil (11) bereitgestellt wird, 50/60 Hz beträgt.

3. Das tragbare PEMFT-Gerät (10) nach Anspruch 1 oder Anspruch 2, wobei eine maximale Spannung, die durch das Hochspannungsnetzteil (31) erzeugt wird, 15000 V beträgt.

4. Das tragbare PEMFT-Gerät (10) nach einem der vorstehenden Ansprüche, wobei der Hochspannungsschalter (33) so konfiguriert ist, den geladenen Hochspannungs-Energiespeicherkondensator (32) mit einer Frequenz von etwa 1-2 Hz kurz zu schließen, um den Stromfluss durch die Magnetspule (34) zur Erzeugung der magnetischen Pulse sicherzustellen.

5. Das tragbare PEMFT-Gerät (10) nach einem der vorstehenden Ansprüche, wobei das Gerät (10) so konfiguriert ist, ein hochfrequentes Magnetfeld mit einer Frequenz im Bereich von etwa 115 bis etwa 135 kHz bereitzustellen.

6. Das tragbare PEMFT-Gerät (10) nach einem der vorstehenden Ansprüche, wobei das Gerät (10) so konfiguriert ist, eine magnetische Flussdichte von etwa 200 bis etwa 1000 G bereitzustellen.

7. Das tragbare PEMFT-Gerät (10) nach einem der vorstehenden Ansprüche, wobei ein maximaler Strom in der Magnetspule (34) 4000 A beträgt.

8. Das tragbare PEMFT-Gerät (10) nach einem der vorstehenden Ansprüche, wobei die Benutzeroberfläche (231) Bedienelemente zum Einstellen von Pulsfrequenz, Intensität und Dauer umfasst und vorzugsweise so konfiguriert ist, Echtzeit-Rückmeldungen zu Behandlungsparametern, zur Reaktion des Patienten auf die Therapie und/oder zu diagnostischen Ergebnissen bereitzustellen.

9. Das tragbare PEMFT-Gerät (10) nach einem der vorstehenden Ansprüche, wobei die Benutzeroberfläche (231) so konfiguriert ist, die Auswahl zwischen drei Parameter-Modi zu ermöglichen, die je nach zu behandelnder Tierart auswählbar sind, wobei ein erster Modus der drei Parameter-Modi Behandlungsparameter für Pferde und andere Tiere ähnlicher Größe festlegt, ein zweiter Modus der drei Parameter-Modi Behandlungsparameter für Hunde und andere Tiere ähnlicher Größe festlegt und ein dritter Modus der drei Parameter-Modi Behandlungsparameter für Katzen und andere Tiere ähnlicher Größe festlegt, wobei die Betriebsparameter des ersten Modus die Erzeugung einer Hochspannung von 15 kV umfassen, die Betriebsparameter des zweiten Modus die Erzeugung einer Hochspannung von 9 kV umfassen und die Betriebsparameter des dritten Modus die Erzeugung einer Hochspannung von 6 kV umfassen.

10. Das tragbare PEMFT-Gerät (10) nach einem der vorstehenden Ansprüche, wobei die Hochspannungsabschirmung (35) aus perforierten Metallblechen besteht, wobei das Metall aus Kupfer, Messing, Nickel, Silber, Stahl und Zinn ausgewählt ist, wobei vorzugsweise die Hochspannungsabschirmung (35) aus perforierten Edelstahlblechen besteht.

11. Das tragbare PEMFT-Gerät (10) nach einem der vorstehenden Ansprüche, wobei die Magnetspule (34) in Form einer Kabelschleife (34) aus einem Hochspannungskabel vorgesehen ist, wobei das Hochspannungskabel ein flexibler verzinnter Kupferleiter ist, der von einer koextrudierten Halbleiterschicht, einer hochwertigen Silikonkautschuk-Isolierung und einem PUR-lackierten Kunstfasergeflecht zur erhöhten Reibungsbeständigkeit umhüllt ist, wobei zwei Enden (342) des Hochspannungskabels, die keinen Schleifenabschnitt der Kabelschleife (34), bilden, mit einem Hochspannungsabschnitt des Geräts (10) verbunden sind, wobei die Kabelschleife (34) so ausgelegt ist, dass sie bei Nichtgebrauch in einem freien Raum des Gehäuses (4) außerhalb der Hochspannungsabschirmung (35) abgelegt wird und der Schleifenabschnitt so gestaltet ist, dass er von einem Bediener berührt und zum Durchführen der Therapie an einem zu behandelnden Tier aus dem Gehäuse (4) entnommen werden kann.

12. Das tragbare PEMFT-Gerät (10) nach einem der vorstehenden Ansprüche, ferner mit zwei Rädern (45) und einem zur Portabilität am Gehäuse befestigten Griff, wobei der Griff vorzugsweise ein Teleskopgriff (46) ist.

13. Das tragbare PEMFT-Gerät (10) nach einem der vorstehenden Ansprüche, ferner mit mindestens einem im Gehäuse (4) zur Belüftung vorgesehenen Lüfter (43).

14. Das tragbare PEMFT-Gerät (10) nach einem der vorstehenden Ansprüche, wobei das Gehäuse (4) eine Abdeckung (44) aufweist, die an einem oberen Teil des Gehäuses (4) drehbar befestigt ist und vorzugsweise mit Hilfe von mindestens einem Riegel (42) am Gehäuse verriegelt werden kann.

15. Das tragbare PEMFT-Gerät (10) nach Anspruch 14, ferner mit einer Anzeige (23), vorzugsweise einem Touch-Display, zur Anzeige der Benutzeroberfläche (231), wobei die Anzeige auf einer Innenfläche der Abdeckung (44) angeordnet ist.

## Revendications

1. Un dispositif portable de thérapie par champ électromagnétique pulsé (PEMFT) (10) destiné au traitement d'animaux tels que chiens, chats, chevaux et animaux de taille similaire, comprenant :
un boîtier (4) contenant :
un condensateur de stockage d'énergie haute tension (32) configuré pour stocker l'énergie utilisée pour générer des impulsions magnétiques ;
une bobine magnétique (34) configurée pour générer les impulsions magnétiques à des fins thérapeutiques ;
une unité d'alimentation haute tension (31) configurée pour fournir de l'énergie au condensateur (32) ;
un commutateur haute tension (33) configuré pour contrôler la libération d'énergie à haute tension destinée à la génération d'impulsions magnétiques ;
une interface utilisateur (231) configurée pour contrôler le fonctionnement de l'appareil (10) ; un contrôleur (21) configuré pour réguler la génération et l'émission des impulsions magnétiques ; et
une unité d'alimentation AC/DC (11) ;
et
un cordon d'alimentation détachable (5) configuré pour connecter l'appareil (10) à une alimentation AC via un coupleur d'appareil afin de fournir l'alimentation pour le fonctionnement de l'appareil (10),
le dispositif (10) étant **caractérisé en ce que** le boîtier (4) est un boîtier en plastique durable et comprend en outre une diode de roue libre (36) pour empêcher que le dispositif (10) soit endommagé par des surtensions, et un blindage haute tension (35) configuré pour prévenir les interférences électromagnétiques et enfermant le condensateur de stockage d'énergie haute tension (32), l'unité d'alimentation haute tension (31), le commutateur haute tension (33), la diode de roue libre (36) et le contrôleur (21).

2. Le dispositif PEMFT portable (10) selon la revendication 1, dans lequel une tension d'entrée fournie par la unité d'alimentation AC/DC (11) est de 100-240 V et une fréquence secteur fournie par la unité d'alimentation AC/DC (11) est de 50/60 Hz.

3. Le dispositif PEMFT portable (10) selon la revendication 1 ou la revendication 2, dans lequel une unité d'alimentation haute tension (31) est de 15000 V.

4. Le dispositif PEMFT portable (10) selon l'une quelconque des revendications précédentes, dans lequel le commutateur haute tension (33) est configuré pour mettre en court-circuit le condensateur de stockage d'énergie haute tension (32) chargé à une fréquence d'environ 1-2 Hz afin d'assurer le passage du courant dans la bobine magnétique (34) pour la génération des impulsions magnétiques.

5. Le dispositif PEMFT portable (10) selon l'une quelconque des revendications précédentes, dans lequel l'appareil (10) est configuré pour fournir un champ magnétique à haute fréquence ayant une fréquence comprise entre environ 115 et 135 kHz.

6. Le dispositif PEMFT portable (10) selon l'une quelconque des revendications précédentes, dans lequel l'appareil (10) est configuré pour fournir une densité de flux magnétique comprise entre environ 200 et 1000 G.

7. Le dispositif PEMFT portable (10) selon l'une quelconque des revendications précédentes, dans lequel un courant maximal dans la bobine magnétique (34) est de 4000 A

8. Le dispositif PEMFT portable (10) selon l'une quelconque des revendications précédentes, dans lequel l'interface utilisateur (231) comprend des commandes pour ajuster la fréquence, l'intensité et la durée des impulsions, et est de préférence configurée pour fournir un retour en temps réel sur les paramètres de traitement, la réponse du patient à la thérapie et/ou les résultats diagnostiques.

9. Le dispositif PEMFT portable (10) selon l'une quelconque des revendications précédentes, dans lequel l'interface utilisateur (231) est configurée pour permettre la sélection entre trois modes de paramètres sélectionnables en fonction de l'espèce animale à traiter, le premier mode des trois modes définissant des paramètres de traitement pour les chevaux et autres animaux de taille similaire, le deuxième mode des trois modes définissant des paramètres de traitement pour les chiens et autres animaux de taille similaire, et le troisième mode des trois modes définissant des paramètres de traitement pour les chats et autres animaux de taille similaire, les paramètres de fonctionnement du premier mode comprenant la génération d'une haute tension de 15 kV, les paramètres de fonctionnement du deuxième mode comprenant la génération d'une haute tension de 9 kV, et les paramètres de fonctionnement du troisième mode comprenant la génération d'une haute tension de 6 kV.

10. Le dispositif PEMFT portable (10) selon l'une quelconque des revendications précédentes, dans lequel le blindage haute tension (35) est constitué de feuilles métalliques perforées, le métal étant choisi parmi le cuivre, le laiton, le nickel, l'argent, l'acier et l'étain, de préférence, le blindage haute tension (35) est constitué de feuilles perforées en acier inoxydable.

11. Le dispositif PEMFT portable (10) selon l'une quelconque des revendications précédentes, dans lequel la bobine magnétique (34) est fournie sous la forme d'une boucle de câble (34) constituée d'un cordon haute tension composé d'un conducteur en cuivre étamé recouvert d'une couche semi-conductrice co-extrudée, d'une isolation en caoutchouc silicone de haute qualité et d'une tresse en fibres synthétiques vernie PUR pour une meilleure résistance au frottement, avec deux extrémités (342) du cordon haute tension, qui ne forment pas la partie en boucle de la boucle de câble (34), se connectant à une section haute tension de l'appareil (10), la boucle de câble (34) étant configurée pour être placée dans un espace libre du boîtier (4) à l'extérieur du blindage haute tension (35) lorsqu'elle n'est pas utilisée, la partie en boucle étant conçue pour être manipulée par un opérateur et sortie du boîtier (4) pour effectuer une thérapie sur un animal dans le besoin.

12. Le dispositif PEMFT portable (10) selon l'une quelconque des revendications précédentes, comprenant en outre deux roues (45) et une poignée fixées au boîtier pour faciliter le transport, de préférence, la poignée est télescopique (46).

13. Le dispositif PEMFT portable (10) selon l'une quelconque des revendications précédentes, comprenant en outre au moins un ventilateur (43) disposé dans le boîtier (4) à des fins de ventilation.

14. Le dispositif PEMFT portable (10) selon l'une quelconque des revendications précédentes, dans lequel le boîtier (4) présente un couvercle (44) relié de manière pivotante à la partie supérieure du boîtier (4) et, de préférence, verrouillable sur le boîtier (4) à l'aide d'au moins un loquet (42).

15. Le dispositif PEMFT portable (10) selon la revendication 14, comprenant en outre un écran (23), de préférence un écran tactile, pour afficher l'interface utilisateur (231), l'écran étant disposé sur une surface intérieure du couvercle (44).
